# EUROPÄISCHE PATENTSCHRIFT

(11) **EP 4 415 767 B1**
(45) Veröffentlichungstag und Bekanntmachung des Hinweises auf die Patenterteilung: **05.11.2025**
(21) Anmeldenummer: 23712218.9
(22) Anmeldetag: 16.03.2023
(51) Int. Cl.: A61L 2/26, A61L 2/07

(54) **STERILISIERBEHÄLTER, STEUERUNGSVORRICHTUNG UND ENTSPRECHENDES SYSTEM**
STERILISING CONTAINER, CONTROL DEVICE AND CORRESPONDING SYSTEM
RÉCIPIENT DE STÉRILISATION, DISPOSITIF DE COMMANDE ET SYSTÈME CORRESPONDANT

(30) Priorität: 17.03.2022 DE 102022106282
(43) Veröffentlichungstag der Anmeldung: 21.08.2024
(73) Patentinhaber: AESCULAP AG, 78532 Tuttlingen (DE)
(72) Erfinder: JANSEN-TROY, Arne, 78333 Stokach (DE); HENKE, Matthias, 78048 Villingen-Schwenningen (DE); KIESSLING, Daniel, 78502 Villingen-Schwenningen (DE)
(74) Vertreter: Winter, Brandl - Partnerschaft mbB
(86) Internationale Anmeldenummer: PCT/EP2023/056815
(87) Internationale Veröffentlichungsnummer: WO 2023/175109

(56) Entgegenhaltungen:
- WO-A1-2014/159696
- WO-A1-2021/072134
- WO-A2-2017/044906
- AU-A1- 2020 248 475
- CN-A- 108 686 235
- US-A1- 2017 096 279
- US-A1- 2017 252 474
- US-A1- 2020 100 858
- US-A1- 2022 028 192
- US-A1- 2022 061 572

## Beschreibung

### Technisches Gebiet

Die vorliegende Offenbarung bezieht sich auf einen Sterilisierbehälter für medizinische Zwecke gemäß dem Oberbegriff des Anspruchs 1, insbesondere auf einen Sterilisierbehälter, dessen Deckel mittels einer Verschlusseinrichtung an einem Bodenelement festlegbar ist, indem eine Steuerungseinrichtung ein Betätigungselement mittels einer in einem Energiespeicher gespeicherten Betätigungsenergie derart steuert, dass ein Verschlusselement betätigt wird. Die vorliegende Offenbarung bezieht sich des Weiteren auf eine Steuerungsvorrichtung zum externen Steuern der Steuerungseinrichtung des Sterilisierbehälters sowie auf ein System mit einem offenbarungsgemäßen Sterilisierbehälter und einer offenbarungsgemäßen Steuerungsvorrichtung.

### Stand der Technik

Ein gattungsgemäßer Sterilisierbehälter ist beispielsweise aus der DE 36 32 674 A1 bekannt und weist ein Bodenelement und einen Deckel auf. Um den Deckel an dem Bodenelement festlegen zu können, weist der gattungsgemäße Sterilisierbehälter eine Verschlusseinrichtung auf, in welcher ein Betätigungselement ein Verschlusselement betätigt, wenn eine Steuerungseinrichtung eine entsprechende in einem Energiespeicher gespeicherte Energie freigibt. Diese Freigabe erfolgt gemäß der DE 36 32 674 A1 infolge von Sensorsignalen von Sensoren, die an dem Sterilisierbehälter angebracht sind.

An dem gattungsgemäßen Sterilisierbehälter ist von Nachteil, dass ein verschlossener Zustand des Sterilisierbehälters nur unmittelbar nach einer ordnungsgemäß in einem Autoklav abgelaufenen Sterilisierung eine Keimfreiheit im Inneren des Sterilisierbehälters indiziert. Um ein späteres Öffnen und damit ein mögliches Verunreinigen eines Innenbereichs des Sterilisierbehälters erkennen zu können, muss an dem Sterilisierbehälter gemäß DE 36 32 674 A1 eine Sicherheitsplombe, beispielsweise gemäß EP 2 896 036 B1, angebracht werden.

Die US 2015 / 0 374 868 A1 offenbart einen Sterilisierbehälter der einen fest mit einer Bodenwanne verbundenen Deckel aufweist, welcher mittels vierer Aktoren relativ zur Bodenwanne verschiebbar ist. Eine Demontage des Deckels von der Bodenwanne kann nur erfolgen, indem der Deckel und die Aktoren voneinander getrennt werden.

In der US 2022 / 0 028 192 A1 wird ein desinfizierbares, berührungsloses Lieferfachsystem offenbart, welches nicht für den medizinischen Gebrauch ausgebildet ist und welches Türen aufweist, die mittels Gelenke festgelegt sind.

Die WO 2014 / 159 696 A1 offenbart einen Sterilisierbehälter mit einem Ventilmechanismus, der eine Leiterplatte aufweist.

In der US 2017 / 0 096 279 A1 wird ein Behälter offenbart, der nicht dazu ausgebildet ist, für medizinische Zwecke bzw. zum Sterilisieren verwendet zu werden. Ein Deckel des offenbarten Behälters ist mit einer Seitenwand über ein Gelenk festgelegt.

Die CN 108 686 235 A offenbart einen Desinfektionsschrank mit intelligenter Steuerung und ein Desinfektionsverfahren dafür. In der US 2020/100858 A1 wird ein Sterilisierbehälter mit Merkmalen zur Abdichtung eines Volumens gegen das Eindringen von Verunreinigungen offenbart. Die US 2022/061572 A1 offenbart eine Vorrichtung und ein Verfahren zur sicheren Zustellung eines Pakets. In der WO 2021/072134 A1 wird ein Schrank, welcher eine kontinuierliche Desinfektionsumgebung für die im Schrank enthaltenen Gegenstände bietet offenbart. Die US 2017/252474 A1 offenbart einen biologischen Indikator-Analysator, welcher eine Vielzahl von Vertiefungen, eine Vielzahl von Organismus-Detektor-Merkmalen und ein Benutzer-Eingabe-Merkmal enthält. In der WO 2017/044906 A2 wird ein Sterilisationsgehäuse offenbart, welches einen oder mehrere Sensoren zur Messung von Eigenschaften innerhalb des Behälters während eines Sterilisationsprozesses umfasst.

### Zusammenfassung der Offenbarung

Vor diesem Hintergrund ist es Aufgabe der vorliegenden Offenbarung, einen Sterilisierbehälter bereit zu stellen, der eine vereinfachte oder verbesserte Überwachung eines Sterilisierbehälter in einem Instrumentenkreislauf ermöglicht.

Diese Aufgabe wird durch einen Sterilisierbehälter gemäß Anspruchs 1, durch eine Steuerungsvorrichtung gemäß Anspruch 11 sowie durch ein entsprechendes System mit einem offenbarungsgemäßen Sterilisierbehälter und einer offenbarungsgemäßen Steuerungsvorrichtung gemäß Anspruch 13 gelöst. Vorteilhafte Weiterbildungen sind Gegenstand der Unteransprüche.

Der offenbarungsgemäße Sterilisierbehälter ist für medizinische Zwecke ausgebildet und eignet sich insbesondere für die Aufnahme von ärztlichen oder chirurgischen Instrumenten. Der Sterilisierbehälter weist ein Bodenelement, einen Deckel, eine Verschlusseinrichtung, einen Energiespeicher und eine Steuerungseinrichtung auf.

Das Bodenelement ist insbesondere wannenförmig ausgebildet.

Die Verschlusseinrichtung weist ein Verschlusselement und ein Betätigungselement auf. Das Verschlusselement ist insbesondere ein riegelförmiges Bauteil an dem Bodenelement oder dem Deckel, welches mit einem entsprechenden Gegenstück an dem anderen Teil des Sterilisierbehälters (Deckel oder Bodenelement) formschlüssig zusammenwirken kann. Das Betätigungselement ist insbesondere ein Elektromotor, der das Verschlusselement bewegen bzw. betätigen kann.

Der Energiespeicher ist insbesondere eine Batterie oder ein Akkumulator. Alternativ kann der Energiespeicher auch ein mechanischer Energiespeicher, beispielsweise in Form einer spannbaren Feder, sein.

Die Steuerungseinrichtung ist ausgebildet, das Betätigungselement mittels einer im Energiespeicher gespeicherten Betätigungsenergie derart steuern zu können, dass das Betätigungselement das Verschlusselement betätigt.

Die Steuerungseinrichtung weist einen Empfänger auf, der ausgebildet ist, Betätigungssignale empfangen zu können, welche ein Betätigen des Betätigungselements veranlassen. Anders ausgedrückt ist die Steuerungseinrichtung ausgebildet, über den Sender Betätigungssignale zu empfangen, auszuwerten und entsprechende Befehle zum Betätigen des Verschlusselements an das Betätigungselement auszugeben. Betätigen kann bedeuten, das Verschlusselement von einer schließenden bzw. verriegelnden Stellung in eine öffnende bzw. entriegelnde Stellung zu bewegen oder von der öffnenden bzw. entriegelnden Stellung in die schließende bzw. verriegelnde Stellung zu bewegen. Die Steuerungseinrichtung weist einen Sender auf, der ausgebildet ist, Statussignale senden bzw. aussenden zu können, welche eine Information über einen Status der Verschlusseinrichtung enthalten.

Offenbarungsgemäß ist die Steuerungseinrichtung ausgebildet, das Betätigungselement mittels einer im Energiespeicher gespeicherten Betätigungsenergie derart steuern zu können, dass das Betätigungselement das Verschlusselement betätigt und dadurch den Deckel an dem Bodenelement festlegt. Insbesondere wird der Deckel dichtend an dem Bodenelement festgelegt, so dass keine gasförmige und/oder flüssige Kontamination in einen Innenbereich des Sterilisierbehälters gelangen kann. Der Sterilisierbehälter ist derart ausgebildet ist, dass der Deckel durch Betätigung des Verschlusselements in einen Zustand gebracht werden kann, in welchem der Deckel werkzeuglos von dem Bodenelement demontiert werden kann.

Anders ausgedrückt ist der Sterilisierbehälter offenbarungsgemäß ausgebildet, den Deckel durch Betätigung des Verschlusselements mittels des Betätigungselements an dem Bodenelement festzulegen. Anders ausgedrückt ist der Sterilisierbehälter offenbarungsgemäß derart ausgebildet, eine Verbindungfähigkeit des Deckels bezüglich des Bodenelements bzw. eine Trennbarkeit des Deckels von dem Bodenelement durch Betätigung des Verschlusselements mittels des Betätigungselements zu bewirken. Anders ausgedrückt ist der Sterilisierbehälter insbesondere derart ausgebildet, dass der Deckel durch Betätigung des Verschlusselements von dem Bodenelement getrennt bzw. demontiert werden kann, insbesondere in einen Zustand gebracht werden kann, in welchem der Deckel werkzeuglos, ohne weitere Demontageschritte, von dem Bodenelement getrennt bzw. demontiert werden kann. Vorzugsweise kann der Deckel allein durch Betätigung des Verschlusselements derart, dass das Verschlusselement von der schließenden bzw. verriegelnden Stellung in die öffnende bzw. entriegelnde Stellung bewegt wird, in einen Zustand gebracht werden, in welchem der Deckel werkzeuglos, insbesondere ohne weitere Demontageschritte, von dem Bodenelement getrennt bzw. demontiert werden kann.

Die Steuerungseinrichtung kann insbesondere ausgebildet sein, die Information über den Status der Verschlusseinrichtung aus einer Historie bzw. Aufzeichnung zuvor stattgefundener Betätigungen des Verschlusselements zu extrahieren. Alternativ oder zusätzlich dazu kann die Verschlusseinrichtung auch einen Verschlusssensor aufweisen, der den Status detektieren kann.

Durch den Sender wird in vorteilhafter Weise eine Schnittstelle bereitgestellt, mittels derer die Überwachung des Sterilisierbehälters vereinfacht bzw. verbessert werden kann. Eine entsprechende Sicherheitsplombe ist nicht mehr nötig.

Gemäß einem Aspekt der Offenbarung können der Empfänger und der Sender der Steuerungseinrichtung integral in Form eines Transceivers ausgebildet sein. Durch die Verwendung eines Transceivers kann der offenbarungsgemäße Sterilisierbehälter kostengünstig realisiert werden.

Gemäß einem Aspekt der Offenbarung kann an einer Außenseite des Sterilisierbehälters ein Taster vorgesehen sein, der ausgebildet ist, infolge seiner Aktivierung ein Betätigungssignal an den Empfänger senden zu können. Insbesondere kann eine Aktivierung des Tasters ein Öffnen bzw. Entriegeln des Verschlusselements durch das Betätigungselement veranlassen. Der Taster kann insbesondere derart ausgebildet sein, dass er ausschließlich zwei Zustände (gedrückt bzw. aktiviert und nicht-gedrückt bzw. nicht-aktiviert) annehmen kann. Das Verschlusselement ist insbesondere derart ausgebildet, dass es in dem Fall, in welchem das Betätigungselement das Verschlusselement nicht betätigt, in der schließenden bzw. verriegelnden Stellung ist. Wird der Taster gedrückt (d.h., aktiviert), steuert die Steuerungseinrichtung das Betätigungselement insbesondere derart, dass das Verschlusselement aus der schließenden bzw. verriegelnden Stellung in die öffnende bzw. entriegelnde Stellung bewegt wird.

Die Verwendung eines Tasters ist gegenüber herkömmlichen, manuell öffenbaren Verschlüssen an Sterilisierbehältern vorteilhaft, da die Bedienung eines Tasters einfacher ist als beispielsweise eine Bedienung einer Übertotpunktverriegelung.

Gemäß einem Aspekt der Offenbarung können an einer Außenseite des Sterilisierbehälters zwei Taster vorgesehen sein, die ausgebildet sind, Betätigungssignale an den Empfänger senden zu können, und kann die Steuerungseinrichtung ausgebildet sein, nur infolge einer synchronen bzw. gleichzeitigen und gleichartigen Aktivierung der beiden Taster das Betätigungselement entsprechend den Betätigungssignalen der Taster zu betätigen.

Gemäß einem Aspekt der Offenbarung können an der Außenseite des Sterilisierbehälters zwei Griffe vorgesehen sein und können die beiden Taster jeweils derart an oder auf einem der Griffe angeordnet sein, dass eine Person, die den Sterilisierbehälter mit ihren Händen an den Griffen ergreift, bei einem Ergreifen mit ihren Händen jeweils einen der beiden Taster aktivieren kann. Insbesondere können die Griffe derart voneinander beabstandet angeordnet sein und/oder können die Taster derart voneinander beabstandet angeordnet sein, dass ein zweihändiges Ergreifen der Griffe und/oder ein zweihändiges Aktivieren der Taster erzwungen wird. Die Griffe und/oder die Taster können insbesondere an dem Deckel vorgesehen sein.

Werden die Taster Griffen zugeordnet, kann die Handhabung des Sterilisierbehälters in vorteilhafter Weise gesteuert werden. Wenn die Griffe an dem Deckel angeordnet sind, kann ein Verkanten des Deckels bei einem Anheben des Deckels verhindert bzw. erschwert werden.

Gemäß einem Aspekt der Offenbarung kann an der Außenseite des Sterilisierbehälters eine optische und/oder akustische Anzeige vorgesehen sein, die ausgebildet ist, die Statussignale des Senders in von Menschen wahrnehmbarer Form anzeigen zu können. Insbesondere kann die Anzeige ausgebildet sein, dynamische (d.h. zeitlich fluktuierende) Ton- und/oder Lichtsignale anzuzeigen.

Durch Vorsehen einer Anzeige kann auf eine Bereitstellung von peripheren Auslesegeräten verzichtet und so die Überwachung des Sterilisierbehälter weiter vereinfacht oder verbessert werden.

Gemäß einem Aspekt der Offenbarung können der bzw. die Taster und die Anzeige integral in Form einer berührungsempfindlichen Anzeige bzw. eines berührungsempfindlichen Bildschirms oder Touchscreens oder mehrerer berührungsempfindlichen Anzeigen bzw. berührungsempfindlicher Bildschirme oder Touchscreens ausgebildet sein.

Durch Vorsehen von berührungsempfindlicher Anzeigen kann die intuitive Bedienung des Sterilisierbehälters ermöglicht bzw. verbessert werden. Außerdem kann die Sterilisierbarkeit bzw. Reinigbarkeit der Taster verbessert werden.

Gemäß einem Aspekt der Offenbarung kann der Empfänger zum kabellosen Empfangen von Betätigungssignalen ausgebildet sein und/oder kann der Sender zum kabellosen Senden von Statussignalen ausgebildet sein. Grundsätzlich kann der Sterilisierbehälter auch derart ausgebildet sein, dass Betätigungssignale und/oder Statussignale über ein oder mehrere Kabel ein- bzw. ausgelesen werden können. Werden der Sender und der Empfänger der Steuerungseinrichtung allerdings für eine kabellose Kommunikation ausgebildet, kann auf Schnittstellen für Kabelstecker verzichtet und somit eine zu reinigende Oberfläche des Sterilisierbehälters in vorteilhafter Weise verringert werden.

Gemäß einem Aspekt der Offenbarung kann die Verschlusseinrichtung ein Notöffnungselement aufweisen, das ausgebildet ist, nur mittels eines Werkzeugs demontierbar zu sein und bei einer alleinigen Demontage des Notöffnungselements die Wirkung des Verschlusselements aufzuheben. Insbesondere kann das Verschlusselement einen Hinterschnitt aufweisen und kann derjenige Abschnitt des Verschlusselements, an dem der Hinterschnitt ausgebildet ist, mit dem restlichen Teil des Verschlusselements über das Notöffnungselement in Form einer Schraube oder eines anderen Verbindungselements verbunden sein. Alternativ kann auch ein Teil des Betätigungselements, der in einem montierten Zustand mit dem Verschlusselement interagieren kann, mit dem restlichen Teil des Betätigungselements über das Notöffnungselement in Form einer Schraube oder eines anderen Verbindungselements verbunden sein. Alternativ kann auch ein Teil des Sterilisierbehälters, der in dem geschlossenen Zustand des Sterilisierbehälters mit dem Verschlusselement in seiner verriegelnden Stellung formschlüssig zusammenwirkt, mit dem restlichen Teil des Sterilisierbehälters über das Notöffnungselement in Form einer Schraube oder eines anderen Verbindungselements verbunden sein. Vorzugsweise ist das Notöffnungselement derart ausgebildet, dass es nur mit einem Schlüssel als Werkzeug demontiert werden kann.

Durch Vorsehen des Notöffnungselements kann im Falle eines Ausfalls der Steuerungseinrichtung ein Zugang zum Inneren des Sterilisierbehälter gewährleistet werden.

Gemäß einem Aspekt der Offenbarung kann der Energiespeicher derart ausgebildet sein, dass er kabellos aufgeladen werden kann. Insbesondere kann der Energiespeicher eine Induktionsspule zum kabellosen Aufladen aufweisen.

Wird der Energiespeicher derart ausgebildet, dass er kabellos aufgeladen werden kann, ist es in vorteilhafter Weise möglich, einen Energiespeicher mit geringem Energiespeichervermögen einzusetzen, ohne Abstriche bei der Funktionalität hinnehmen zu müssen.

Eine offenbarungsgemäße Steuerungsvorrichtung ist geeignet, eine Steuerungseinrichtung eines offenbarungsgemäßen Sterilisierbehälters extern zu steuern. "Extern" heißt insbesondere, dass die Steuerungsvorrichtung nicht Bestandteil des Sterilisierbehälters ist oder nicht fest mit dem Sterilisierbehälter verbunden ist.

Die offenbarungsgemäße Steuerungsvorrichtung weist einen Sender auf (Sender der Steuerungsvorrichtung im Gegensatz zum Sender der Steuerungseinrichtung), der ausgebildet ist, Betätigungssignale an die Steuerungseinrichtung senden zu können, und weist einen Empfänger auf (Empfänger der Steuerungsvorrichtung im Gegensatz zum Empfänger der Steuerungseinrichtung), der ausgebildet ist, Statussignale von der Steuerungseinrichtung empfangen zu können. Vorzugsweise sind der Sender und der Empfänger der Steuerungsvorrichtung integral in Form eines Transceivers ausgebildet. Insbesondere ist die Steuerungsvorrichtung ausgebildet, kabellos mit der Steuerungseinrichtung des Sterilisierbehälter kommunizieren zu können. Anders ausgedrückt, ist die Steuerungsvorrichtung insbesondere als eine Fernsteuerung für den Sterilisierbehälter ausgebildet. Insbesondere ist die externe Steuerungsvorrichtung an oder in einem Autoklav ausgebildet.

Wird eine externe Steuerungsvorrichtung bereitgestellt, kann der Automatisierungsgrad eines Sterilisiervorganges erhöht werden.

Gemäß einem Aspekt der Offenbarung kann die Steuerungsvorrichtung einen Temperatursensor, einen Drucksensor und/oder einen Feuchtigkeitssensor aufweisen und kann ausgebildet sein, bei Messung einer vorbestimmten Temperatur, eines vorbestimmten Drucks und/oder einer vorbestimmten Feuchtigkeit oder bei Erreichen einer vorbestimmten Anzahl wiederholter Messungen der vorbestimmten Temperatur, des vorbestimmten Drucks, und/oder der vorbestimmten Feuchtigkeit ein Betätigungssignal auszusenden, insbesondere an die Steuerungseirichtung des Sterilisierbehälters zu senden.

Werden die Sensoren in einer Fernsteuerung angeordnet, können die Herstellungskosten für den Sterilisierbehälter verringert werden.

Ein offenbarungsgemäßes System weist einen offenbarungsgemäßen Sterilisierbehälter und eine offenbarungsgemäße Steuerungsvorrichtung auf. Die Steuerungseinrichtung des Sterilisierbehälters des offenbarungsgemäßen Systems ist ausgebildet, feststellen zu können, ob sich die Steuerungsvorrichtung in einem vorbestimmten Bereich um den Sterilisierbehälter befindet. Des Weiteren ist die Steuerungseinrichtung ausgebildet, nur ein Betätigungssignal an die Betätigungseinrichtung senden zu können, solange sich die Steuerungsvorrichtung in dem vorbestimmten Bereich um den Sterilisierbehälter befindet. Die Feststellung, ob sich die Steuerungsvorrichtung in einem vorbestimmten Bereich um den Sterilisierbehälter befindet, kann anhand einer in der Steuerungseinrichtung ablaufenden Auswertung von Signalen, die der Empfänger der Steuerungseinrichtung von dem Sender der Steuerungsvorrichtung empfängt, erfolgen. Insbesondere kann die Feststellung anhand der Signalstärke dieser Signale erfolgen.

Wird eine Betätigung des Betätigungselements nur zugelassen, wenn sich der Sterilisierbehälter in der Nähe der Steuerungsvorrichtung, d.h. in einem vorbestimmten Bereich um die Steuerungsvorrichtung, befindet, kann ein ordnungsgemäßer Ablauf einer Sterilisierung erleichtert werden.

### Kurzbeschreibung der Zeichnungen

Die vorliegende Erfindung wird im Folgenden anhand eines bevorzugten Ausführungsbeispiels unter Bezugnahme auf die beigefügten Zeichnungen näher beschrieben. Es zeigen:
Fig. 1 eine Seitenansicht eines offenbarungsgemäßen Sterilisierbehälters, und
Fig. 2 eine Querschnittsansicht des offenbarungsgemäßen Sterilisierbehälters entsprechend der Linie II-II der Fig. 1.

### Detaillierte Beschreibung bevorzugter Ausführungsformen

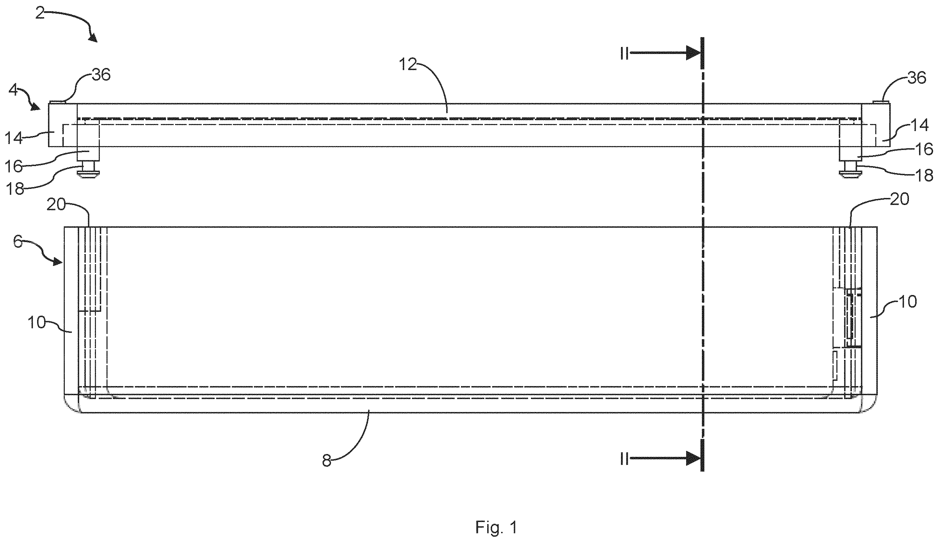

Fig. 1 zeigt eine Seitenansicht einer offenbarungsgemäßen Sterilisierbehälters 2 mit einem Deckel 4 und einem Bodenelement 6. Das Bodenelement 6 ist wannenförmig und weist einen Boden 8 mit einem im Wesentlichen rechteckigen Umriss und vier sich von den vier Kanten des Bodens 8 aus erstreckende, im Wesentlichen rechteckige Seitenwände 10 auf.

Der Deckel 4 weist eine Deckteil 12 mit einem im Wesentlichen rechteckigen Umriss und vier sich von den vier Kanten des Deckteils 12 aus erstreckende, im wesentlichen reckeckige Ränder 14 auf.

Der Deckel 4 und die Ränder 14 sind derart dimensioniert, dass freie Kanten der Seitenwände 10 über eine (nicht gezeigte) Dichtung mit dem Deckteil 12 und/oder den Rändern 14 des Deckels 4 in Kontakt stehen, wenn der Deckel 4 in einem geschlossenen Zustand des Sterilisierbehälters 2 auf das Bodenelement 6 aufgesetzt ist. In dem geschlossenen Zustand des Sterilisierbehälters 2 überlappen die Ränder 14 die freien Kanten der Seitenwände 10. Die Dichtung des Sterilisierbehälters ist derart ausgestaltet, dass in dem geschlossenen Zustand des Sterilisierbehälters 2 flüssige und/oder gasförmige Kontaminationen nicht in einen von dem Boden 8, dem Deckteil 12, den Seitenwänden 10 und den Rändern 14 definierten Innenbereich eindringen können.

Um den Deckel 4 im geschlossenen Zustand des Sterilisierbehälters 2 festzulegen, sind an einer zum Innenbereich weisenden Seite des Deckteils 12 Stifte 16 vorgesehen. Die Stifte 16 erstrecken sich von einem Bereich des Deckteils 12 innerhalb der Ränder 14 aus, an welchem in dem geschlossenen Zustand des Sterilisierbehälters 2 die freien Kanten der Seitenwände über die (nicht gezeigte) Dichtung an dem Deckteil 12 aufliegen. An den freien Enden der Stifte 16 ist jeweils ein Hinterschnitt in Form einer Umfangsnut 18 vorgesehen, so dass die freien Enden der Stifte 16 pilzförmig sind.

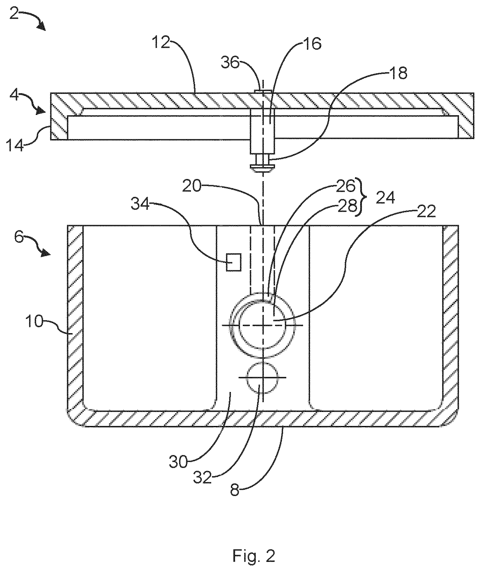

Der Deckel 4 weist zwei Stifte 16 auf, die jeweils mittig an einem der kurzen Ränder 14 angeordnet sind (siehe auch Fig. 2).

Entsprechend den Stiften 16 sind an den freien Rändern der Seitenwände 10, genauer gesagt an den kurzen Seitenwänden 10, jeweils eine Stiftaufnahme 20 vorgesehen, in welche die Stifte 16 in dem geschlossenen Zustand des Sterilisierbehälters 2 aufgenommen sind.

Das Zusammenwirken der Stifte 16 mit dem Bodenelement 6 wird nachfolgend nur bezüglich eines Stiftes 16 erläutert. Der Sterilisierbehälter 2 ist derart symmetrisch ausgebildet, dass diese Erläuterungen entsprechend auch für den anderen Stift 16 gelten.

Fig. 2 zeigt eine Querschnittsansicht des Sterilisierbehälters 2 entsprechend der Linie II-II der Fig. 1.

Wie in Fig. 2 gezeigt, ist an einem dem entsprechenden freien Rand der Seitenwand 10 abgewandten Ende der Stiftaufnahme 20 ein Verschlusselement 22 in Form eines Exzenters vorgesehen, welches mittels eines (nicht gezeigten) Aktors (beispielsweise eines Elektromotors) bewegt bzw. betätigt werden kann. Der Aktor stellt somit ein offenbarungsgemäßes Betätigungselement dar. Der Aktor kann mithilfe eines (nicht gezeigten) Energiespeichers betrieben werden.

Das Verschlusselement 22 weist eine Nockenscheibe 24 auf, deren Nocken 26 auf einer axialen Seite bzw. Stirnfläche einer Scheibe 28 mit rundem Umriss angeordnet ist. Der Nocken 26 erstreckt sich entlang eines Bereichs (etwa ein Viertel) des Umfangs der Scheibe 28 und weist einen entlang des Umfangs der Scheibe 28 verlaufenden, keilförmigen Umriss auf. Das Verschlusselement 22 ist in der entsprechenden Seitenwand 10 derart an der Stiftaufnahme 20 angeordnet, dass sich die Scheibe 28 parallel zu der Seitenwand 10 erstreckt und der Nocken 26 wahlweise in die oder aus der Stiftaufnahme 20 bewegt werden kann.

Wenn der Stift 16 in dem geschlossenen Zustand des Sterilisierbehälters 2 in der Stiftaufnahme 20 aufgenommen ist, kann das Verschlusselement 22 mittels des Aktors derart bewegt bzw. gedreht werden, dass der Nocken 26 in einer verriegelnden Stellung (siehe Fig. 2) in die Umfangsnut 18 des Stifts 16 eingreift und damit eine Relativbewegung des Stifts 16 aus der Stiftaufnahme 20 verhindert. Um den Stift 16 freizugeben, kann das Verschlusselement 22 derart gedreht werden, dass der Nocken 26 in einer öffnenden Stellung nicht in die Stiftaufnahme eingreift und somit auch nicht mit der Umfangsnut 18 des Stifts 16 formschlüssig zusammenwirken kann.

Aufgrund der keilförmigen Ausbildung des Nockens 26 kann durch Betätigen bzw. Bewegen bzw. Drehen des Verschlusselements 22 eine Relativbewegung des Stifts 16 entlang der Erstreckungsrichtung der Stiftaufnehme 20 hervorgerufen werden, wenn der Stift 16 in der Stiftaufnahme 20 aufgenommen ist. Somit ist es möglich, den Deckel 4 mithilfe des Verschlusselements 22 anzuheben bzw. die (nicht gezeigte) Dichtung weniger stark zu beanspruchen und den Sterilisierbehälter 2 von dem geschlossenen Zustand in einen geöffneten bzw. undichten Zustand zu überführen.

Um den in der entsprechenden Seitenwand 10 integrierten (nicht gezeigten) Aktor steuern zu können, ist eine Steuerungseinrichtung 30 ebenfalls in der entsprechenden Seitenwand 10 integriert. Die Steuerungseinrichtung 30 weist einen Sensor 32 auf, welcher einen Druck, eine Temperatur und/oder eine Feuchtigkeit im Innenbereich des Sterilisierbehälters 2 messen kann.

Offenbarungsgemäß ist die Steuerungseinrichtung 30 mit einem Transceiver 34 ausgestattet. An einer Außenseite des Deckels 4 sind an Randbereichen Taster 36 vorgesehen (siehe auch Fig. 1). Die Taster 36 sind derart ausgebildet, dass diese ein Betätigungssignal aussenden können, wenn sie gedrückt werden. Die Steuerungseinrichtung 30 ist derart ausgebildet, dass sie den (nicht gezeigten) Aktor betätigt, wenn der Transceiver 34 von beiden Tastern 36 übereinstimmende Betätigungssignale empfängt. Die Steuerungseinrichtung 30 ist ausgebildet, über den Transceiver 34 den Status des Verschlusselements 22 bzw. den Status der durch den Stift 16, der Stiftaufnahme 20, das Verschlusselement 22 und den (nicht gezeigten) Aktor gebildeten Verschlusseinrichtung mittels Statussignale auszusenden.

Offenbarungsgemäß ist die Steuerungseinrichtung 30 auch ausgebildet, über den Transceiver 34 nicht nur von den Tastern 36, sondern auch von einer (nicht gezeigten) externen Steuerungsvorrichtung Betätigungssignale zum Betätigen des Verschlusselements 22 empfangen zu können.

### Bezugszeichenliste

- 2: Sterilisierbehälter
- 4: Deckel
- 6: Bodenelement
- 8: Boden
- 10: Seitenwand
- 12: Deckteil
- 14: Rand des Deckels
- 16: Stift
- 18: Umfangsnut
- 20: Stiftaufnahme
- 22: Verschlusselement
- 24: Nockenscheibe
- 26: Nocken
- 28: Scheibe
- 30: Steuerungseinrichtung
- 32: Sensor
- 34: Transceiver
- 36: Taster

## Patentansprüche

1. Sterilisierbehälter (2) für medizinische Zwecke, mit
einem Bodenelement (6),
einem Deckel (4),
einer Verschlusseinrichtung, die ein Verschlusselement (22) und ein Betätigungselement aufweist,
einem Energiespeicher und
einer Steuerungseinrichtung (30), die ausgebildet ist, das Betätigungselement mittels einer im Energiespeicher gespeicherten Betätigungsenergie derart steuern zu können, dass das Betätigungselement das Verschlusselement (22) betätigt, wobei die Steuerungseinrichtung (30) einen Empfänger aufweist, der ausgebildet ist, Betätigungssignale empfangen zu können, welche ein Betätigen des Betätigungselements veranlassen, und die Steuerungseinrichtung (30) einen Sender aufweist, der ausgebildet ist, Statussignale senden zu können, welche eine Information über einen Status der Verschlusseinrichtung enthalten,
**dadurch gekennzeichnet, dass**
der Sterilisierbehälter (2) ausgebildet ist, den Deckel (4) durch Betätigung des Verschlusselements (22) mittels des Betätigungselements an dem Bodenelement (6) festzulegen, wobei das Verschlusselement (22) in Form eines Exzenters vorgesehen ist.

2. Sterilisierbehälter (2) nach Anspruch 1, **dadurch gekennzeichnet, dass** der Empfänger und der Sender der Steuerungseinrichtung integral in Form eines Transceivers (34) ausgebildet sind.

3. Sterilisierbehälter (2) nach Anspruch 1 oder 2, **dadurch gekennzeichnet, dass** an einer Außenseite des Sterilisierbehälters (2) ein Taster (36) vorgesehen ist, der ausgebildet ist, infolge seiner Aktivierung ein Betätigungssignal an den Empfänger senden zu können.

4. Sterilisierbehälter (2) nach Anspruch 1 oder 2, **dadurch gekennzeichnet, dass** an einer Außenseite des Sterilisierbehälters (2) zwei Taster (36) vorgesehen sind, die ausgebildet sind, Betätigungssignale an den Empfänger senden zu können, und die Steuerungseinrichtung ausgebildet ist, nur infolge einer gleichzeitigen und gleichartigen Aktivierung der beiden Taster (36) das Betätigungselement entsprechend den Betätigungssignalen der Taster (36) zu betätigen.

5. Sterilisierbehälter (2) nach Anspruch 4, **dadurch gekennzeichnet, dass** an der Außenseite des Sterilisierbehälters (2) zwei Griffe vorgesehen sind und die beiden Taster (36) jeweils derart an oder auf einem der Griffe angeordnet sind, dass eine Person, die den Sterilisierbehälter (2) mit ihren Händen an den Griffen ergreift, bei einem Ergreifen mit ihren Händen jeweils einen der beiden Taster (36) aktivieren kann.

6. Sterilisierbehälter (2) nach einem der Ansprüche 1 bis 5, **dadurch gekennzeichnet, dass** an der Außenseite des Sterilisierbehälters (2) eine optische und/oder akustische Anzeige vorgesehen ist, die ausgebildet ist, die Statussignale des Senders in von Menschen wahrnehmbarer Form anzeigen zu können.

7. Sterilisierbehälter (2) nach Anspruch 6 und einem der Ansprüche 2 bis 5, **dadurch gekennzeichnet, dass** der/die Taster (36) und die Anzeige integral in Form einer berührungsempfindlichen Anzeige oder mehrerer berührungsempfindlichen Anzeigen ausgebildet sind.

8. Sterilisierbehälter (2) nach einem der Ansprüche 1 bis 7, **dadurch gekennzeichnet, dass** der Empfänger zum kabellosen Empfangen von Betätigungssignalen und/oder der Sender zum kabellosen Senden von Statussignalen ausgebildet sind/ist.

9. Sterilisierbehälter (2) nach einem der Ansprüche 1 bis 8 **dadurch gekennzeichnet, dass** die Verschlusseinrichtung ein Notöffnungselement in Form einer Schraube oder eines anderen Verbindungselements aufweist, wobei das Verschlusselement (22) einen Hinterschnitt aufweist und derjenige Abschnitt des Verschlusselements (22), an dem der Hinterschnitt ausgebildet ist, mit dem restlichen Teil des Verschlusselements über das Notöffnungselement verbunden ist, das ausgebildet ist, nur mittels eines Werkzeugs demontierbar zu sein und bei einer alleinigen Demontage des Notöffnungselements die Wirkung des Verschlusselements aufzuheben.

10. Sterilisierbehälter (2) nach einem der Ansprüche 1 bis 9, **dadurch gekennzeichnet, dass** der Energiespeicher derart ausgebildet ist, dass er kabellos aufgeladen werden kann.

11. System aus einem Sterilisierbehälter (2) nach einem der Ansprüche 1 bis 10 und einer Steuerungsvorrichtung zum externen Steuern der Steuerungseinrichtung (30) des Sterilisierbehälters (2), **dadurch gekennzeichnet, dass** die Steuerungsvorrichtung einen Sender aufweist, der ausgebildet ist, Betätigungssignale an die Steuerungseinrichtung (30) senden zu können, und einen Empfänger aufweist, der ausgebildet ist, Statussignale von der Steuerungseinrichtung (30) empfangen zu können.

12. System nach Anspruch 11, **dadurch gekennzeichnet, dass** die Steuerungsvorrichtung einen Temperatursensor, einen Drucksensor und/oder einen Feuchtigkeitssensor aufweist und ausgebildet ist, bei Messung einer vorbestimmten Temperatur, eines vorbestimmten Drucks und/oder einer vorbestimmten Feuchtigkeit oder bei Erreichen einer vorbestimmten Anzahl wiederholter Messungen der vorbestimmten Temperatur, des vorbestimmten Drucks, und/oder der vorbestimmten Feuchtigkeit ein Betätigungssignal auszusenden.

13. System nach Anspruch 11 oder 12, **dadurch gekennzeichnet, dass** die Steuerungseinrichtung (30) des Sterilisierbehälters (2) ausgebildet ist, feststellen zu können, ob sich die Steuerungsvorrichtung in einem vorbestimmten Bereich um den Sterilisierbehälter (2) befindet, und nur ein Betätigungssignal an die Betätigungseinrichtung senden zu können, solange sich die Steuerungsvorrichtung in dem vorbestimmten Bereich um den Sterilisierbehälter (2) befindet.

## Claims

1. A sterilization container (2) for medical purposes, having
a bottom element (6),
a lid (4),
a closure apparatus comprising a closure element (22) and an actuation element,
an energy storage and
a control apparatus (30) which is configured to be able to control the actuation element via actuation energy stored in the energy storage such that the actuation element actuates the closure element (22), wherein the control apparatus (30) has a receiver which is configured to be able to receive actuation signals which cause actuation of the actuation element, and the control apparatus (30) has a transmitter which is configured to be able to transmit status signals which contain information about a status of the closure apparatus,
**characterized in that**
the sterilization container (2) is configured to fix the lid (4) to the bottom element (6) by actuating the closure element (22) via the actuation element, wherein the closure element (22) is provided in the form of an eccentric.

2. The sterilization container (2) according to claim 1, **characterized in that** the receiver and the transmitter of the control apparatus are integrally configured in the form of a transceiver (34).

3. The sterilization container (2) according to claim 1 or 2, **characterized in that** a button (36) is provided on an outer side of the sterilization container (2), wherein the button (36) is configured to be able to send an actuation signal to the receiver as a result of its activation.

4. The sterilization container (2) according to claim 1 or 2, **characterized in that** two buttons (36) are provided on an outer side of the sterilization container (2), which are configured to be able to transmit actuation signals to the receiver, and the control apparatus is configured to actuate the actuation element according to the actuation signals of the buttons (36) only as a result of a simultaneous and alike activation of the two buttons (36).

5. The sterilization container (2) according to claim 4, **characterized in that** two handles are provided on the outer side of the sterilization container (2), and the two buttons (36) are each arranged at or on one of the handles in such a way that a person gripping the sterilization container (2) with his/her hands on the handles can activate one of the two buttons (36) in each case when gripping it with his/her hands.

6. The sterilization container (2) according to one of claims 1 to 5, **characterized in that** an optical and/or acoustic display, which is configured to be able to display the status signals of the transmitter in a form that can be perceived by humans, is provided on the outer side of the sterilization container (2).

7. The sterilization container (2) according to claim 6 and one of claims 2 to 5, **characterized in that** the button/buttons (36) and the display are integrally configured in the form of a touch-sensitive display or several touch-sensitive displays.

8. The sterilization container (2) according to one of claims 1 to 7, **characterized in that** the receiver is configured to receive actuation signals wirelessly and/or the transmitter is configured to transmit status signals wirelessly.

9. The sterilization container (2) according to one of claims 1 to 8, **characterized in that** the closure apparatus has an emergency opening element in the form of a screw or of another connecting element, wherein the closure element (22) has an undercut and the portion of the closure element (22) at which the undercut is formed is connected to the remaining part of the closure element via the emergency opening element, which is configured to be removable only via a tool and to cancel the effect of the closure element when the emergency opening element alone is disassembled.

10. The sterilization container (2) according to one of claims 1 to 9, **characterized in that** the energy storage is configured to be charged wirelessly.

11. A system comprising a sterilization container (2) according to one of claims 1 to 10 and a control device for externally controlling the control apparatus (30) of the sterilization container (2), **characterized in that** the control device comprises a transmitter which is configured to be able to transmit actuation signals to the control apparatus (30), and has a receiver which is configured to be able to receive status signals from the control apparatus (30).

12. The system according to claim 11, **characterized in that** the control device comprises a temperature sensor, a pressure sensor and/or a humidity sensor and is configured to emit an actuation signal when a predetermined temperature, a predetermined pressure and/or a predetermined humidity is measured or when reaching a predetermined number of repeated measurements of the predetermined temperature, of the predetermined pressure and/or of the predetermined humidity.

13. The system according to claim 11 or 12, **characterized in that** the control apparatus (30) of the sterilization container (2) is configured to be able to determine whether the control device is located in a predetermined region around the sterilization container (2), and to be able to transmit an actuation signal to the actuating apparatus only as long as the control device is located in the predetermined region around the sterilization container (2).

## Revendications

1. Récipient de stérilisation (2) à usage médical, avec
un élément de fond (6),
un couvercle (4),
un dispositif de fermeture qui présente un élément de fermeture (22) et un élément d'actionnement,
un accumulateur d'énergie et
un dispositif de commande (30) qui est formé afin de pouvoir commander l'élément d'actionnement au moyen d'une énergie d'actionnement enregistrée dans l'accumulateur d'énergie de telle manière que l'élément d'actionnement actionne l'élément de fermeture (22), dans lequel le dispositif de commande (30) présente un récepteur qui est formé afin de pouvoir recevoir des signaux d'actionnement qui provoquent un actionnement de l'élément d'actionnement, et le dispositif de commande (30) présente un émetteur qui est formé afin de pouvoir émettre des signaux de statut qui contiennent une information sur un statut du dispositif de fermeture,
**caractérisé en ce que**
le récipient de stérilisation (2) est formé afin de fixer le couvercle (4) par actionnement de l'élément de fermeture (22) au moyen de l'élément d'actionnement à l'élément de fond (6), dans lequel l'élément de fermeture (22) est prévu sous la forme d'une excentrique.

2. Récipient de stérilisation (2) selon la revendication 1, **caractérisé en ce que** le récepteur et l'émetteur du dispositif de commande sont formés d'un seul tenant sous la forme d'un émetteur-récepteur (34).

3. Récipient de stérilisation (2) selon la revendication 1 ou 2, **caractérisé en ce qu'** une touche (36) est prévue sur un côté extérieur du récipient de stérilisation (2), touche qui est formée afin de pouvoir envoyer à la suite de son activation un signal d'actionnement à l'émetteur.

4. Récipient de stérilisation (2) selon la revendication 1 ou 2, **caractérisé en ce que** deux touches (36) sont prévues sur un côté extérieur du récipient de stérilisation (2), lesquelles sont formées afin de pouvoir émettre des signaux d'actionnement à l'émetteur, et le dispositif de commande est formé afin d'actionner uniquement à la suite d'une activation simultanée et identique des deux touches (36) l'élément d'actionnement selon les signaux d'actionnement des touches (36).

5. Récipient de stérilisation (2) selon la revendication 4, **caractérisé en ce que** deux prises sont prévues sur le côté extérieur du récipient de stérilisation (2) et les deux touches (36) sont agencées respectivement au niveau ou sur une des prises de telle manière qu'une personne qui saisit le récipient de stérilisation (2) avec ses mains au niveau de prises, puisse activer respectivement l'une des deux touches (36) lors d'une saisie avec ses mains.

6. Récipient de stérilisation (2) selon l'une quelconque des revendications 1 à 5, **caractérisé en ce qu'**un affichage optique et/ou acoustique est prévu sur le côté extérieur du récipient de stérilisation (2), affichage qui est formé afin de pouvoir afficher les signaux de statut de l'émetteur sous une forme perceptible par des hommes.

7. Récipient de stérilisation (2) selon la revendication 6 et l'une quelconque des revendications 2 à 5, **caractérisé en ce que** la/les touches (36) et l'affichage sont présents d'un seul tenant sous la forme d'un affichage tactile ou de plusieurs affichages tactiles.

8. Récipient de stérilisation (2) selon l'une quelconque des revendications 1 à 7, **caractérisé en ce que** le récepteur est formé pour la réception sans câble de signaux d'actionnement et/ou l'émetteur est formé pour l'émission sans câble de signaux de statut.

9. Récipient de stérilisation (2) selon l'une quelconque des revendications 1 à 8 **caractérisé en ce que** le dispositif de fermeture présente un élément d'ouverture d'urgence sous la forme d'une vis ou d'un autre élément de liaison, dans lequel l'élément de fermeture (22) présente une contre-dépouille et la section de l'élément de fermeture (22) sur laquelle est formée la contre-dépouille, est reliée à la partie restante de l'élément de fermeture par le biais de l'élément d'ouverture d'urgence qui est formé afin de pouvoir être démonté uniquement au moyen d'un outil et de lever l'action de l'élément de fermeture lors d'un seul démontage de l'élément d'ouverture d'urgence.

10. Récipient de stérilisation (2) selon l'une quelconque des revendications 1 à 9, **caractérisé en ce que** l'accumulateur d'énergie est formé de telle manière qu'il puisse être chargé sans câble.

11. Système avec un récipient de stérilisation (2) selon l'une quelconque des revendications 1 à 10 et un appareil de commande pour la commande externe de l'appareil de commande (30) du récipient de stérilisation (2), **caractérisé en ce que** l'appareil de commande présente un émetteur qui est formé afin de pouvoir envoyer des signaux d'actionnement à l'appareil de commande (30) et présente un récepteur qui est formé afin de pouvoir recevoir des signaux de statut de l'appareil de commande (30).

12. Système selon la revendication 11, **caractérisé en ce que** l'appareil de commande présente un capteur de température, un capteur de pression et/ou un capteur d'humidité et est formé afin d'émettre un signal d'actionnement lors de la mesure d'une température prédéterminée, d'une pression prédéterminée et/ou d'une humidité prédéterminée ou lors de l'atteinte d'un nombre prédéterminé de mesures répétées de la température prédéterminée, de la pression prédéterminée et/ou de l'humidité prédéterminée.

13. Système selon la revendication 11 ou 12, **caractérisé en ce que** l'appareil de commande (30) du récipient de stérilisation (2) est formé afin de pouvoir constater si l'appareil de commande se trouve dans une zone prédéterminée autour du récipient de stérilisation (2), et de pouvoir envoyer uniquement un signal d'actionnement au dispositif d'actionnement tant que le dispositif de commande se trouve dans la zone prédéterminée autour du récipient de stérilisation (2).
